# EUROPÄISCHE PATENTSCHRIFT

(11) **EP 1 355 915 B1**
(45) Veröffentlichungstag und Bekanntmachung des Hinweises auf die Patenterteilung: **10.08.2005**
(21) Anmeldenummer: 02708318.7
(22) Anmeldetag: 28.01.2002
(51) Int. Cl.: C07H 19/06

(54) **MARKIERUNGSFÄHIGE VERBINDUNGEN ZUR EINFACHEN SYNTHESE VON 3'-[18F]FLUOR-3'-DEOXYTHYMIDIN UND VERFAHREN ZU DEREN HERSTELLUNG**
LABELLING COMPOUNDS FOR THE SIMPLE SYNTHESIS OF 3'(18F)FLUORO-3'-DEOXY THYMIDINE AND A METHOD FOR THE PRODUCTION THEREOF
COMPOSES CAPABLES DE MARQUAGE POUR LA SYNTHESE DE 3' 18F -FLUOR-3'-DESOXYTHYMIDINE ET PROCEDE DE PREPARATION DESDITS COMPOSES

(30) Priorität: 31.01.2001 DE 10104250
(43) Veröffentlichungstag der Anmeldung: 29.10.2003
(73) Patentinhaber: Deutsches Krebsforschungszentrum Stiftung des öffentlichen Rechts, 69120 Heidelberg (DE)
(72) Erfinder: MARTIN, Stefan, Johannes, 69221 Dossenheim (DE); EISENBARTH, Joseph, Antonius, Maria, 68775 Ketsch (DE); WAGNER-UTERMANN, Ulrike, 64646 Heppenheim (DE); EISENHUT, Michael, 69118 Heidelberg (DE); MIER, Walter, 69115 Heidelberg (DE)
(74) Vertreter: Störle, Christian, Dr.
(86) Internationale Anmeldenummer: PCT/EP2002/000865
(87) Internationale Veröffentlichungsnummer: WO 2002/060913

(56) Entgegenhaltungen:
- EISENBARTH, J.A. ET AL.: "PS 838. PRECURSORS OF [18F]FLT: DEVELOPMENT OF SIMPLY SYNTHETIC PATHWAYS" EUROPEAN JOURNAL OF NUCLEAR MEDICINE, Bd. 27, Nr. 8, 2000, Seite 1220 XP002199400 in der Anmeldung erwähnt
- GRIERSON, J.R. ET AL.: "DEVELOPMENTS IN THE RADIOSYNTHESIS OF [F-18]FLT" JOURNAL OF NUCLEAR MEDICINE, Bd. 39, Nr. 5, 1998, Seite 22P XP008003383
- GRIERSON, J.R. ET AL.: "RADIOSYNTHESIS OF 3'-DEOXY-3'-[18F]FLUOROTHYMIDINE: [18F]FLT FOR IMAGING OF CELLULAR PROLIFERATION IN VIVO" NUCLEAR MEDICINE & BIOLOGY, Bd. 27, 2000, Seiten 143-156, XP002199401 in der Anmeldung erwähnt

## Beschreibung

Die Erfindung bezieht sich auf Verbindungen, die als markierungsfähige Vorläufersubstanzen zur Herstellung von 3'-[¹⁸F]Fluor-3'-deoxythymidin ([¹⁸F]FLT) geeignet sind, Verfahren zu deren Herstellung und deren Verwendung zur Synthese von [¹⁸F]FLT.

In der Nuklearmedizin können mit Hilfe der sogenannten Positronen-Emissions-Tomographie (PET) Wirkungsweisen von körpereigenen oder -fremden Stoffen (Gifte, Pharmaka) sowie Stoffwechselvorgänge im Gehim und anderen Organen, insbesondere in Tumoren, untersucht werden. Gerade in der Tumordiagnostik wird PET häufig eingesetzt. Dazu werden biologisch aktive, mit Positronenemittierenden Radionukliden markierte Verbindungen injiziert, und die emittierte γ-Strahlung in Tomogrammen erfaßt.

Als markierte Verbindung kann dabei 3'-[¹⁸F]Fluor-3'-deoxythymidin([¹⁸F]FLT) 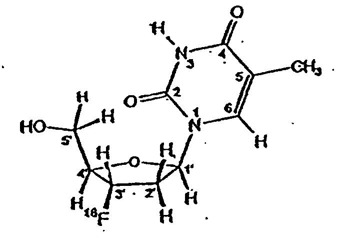 eingesetzt werden, die sich in schnell teilenden Zellen, wie sie bei Tumoren vorliegen, anreichert. Auf diese Weise lassen sich zum Beispiel Tumore, besonders im Hirn, aber auch im Körperstamm, auffinden oder therapiebegleitend das Ansprechen auf ein Behandlungsschema oder dessen Optimierung beurteilen.

Die Herstellung von [¹⁸F]FLT verläuft bisher über mehrere Zwischenstufen, bei denen hochtoxische Chemikalien eingesetzt werden oder entstehen, die dann vor der Verabreichung des Proliferationsmarkers [¹⁸F]FLT vollständig abgetrennt werden müssen.

So beschreiben Grierson J. R. und Shields A. F. in *Nucl. Med. Biol*., 2000, 27, 143 - 156 eine aufwendige Synthese von Markierungsvorläufern für die Herstellung von [¹⁸F]FLT, wobei zum Teil hochtoxische Verbindungen, wie Phosgen, eingesetzt werden. Dabei wird mit Dimethoxybenzyl-*N*-Schutzgruppen gearbeitet, die oxidativ mit Cer(IV)-Ammoniumnitrat abgespaltet werden müssen. Cer-Verbindungen sind aber toxisch, weshalb sie in einer reproduzierbaren Fällungsreaktion quantitativ abgetrennt werden müssen. Diese Arbeitsabläufe müssen manuell durchgeführt werden, was aber aus Strahlenschutzgründen wiederum vermieden werden soll, weil die Strahlenbelastung und die Gefahr der Kontamination von Menschen so niedrig wie möglich gehalten werden soll.

Machulla et al. in *J. Radioanal. Nucl. Chem*., 2000, *243*, 843 - 846 und Wodarski et al. in *J. Labelled Cpd. Radiopharm*, 2000, 43, 1211 - 1218 beschreiben die Synthese von Markierungsvorläufern für die Herstellung von [¹⁸F]FLT, wobei aber extreme Reaktionsbedingungen notwendig sind, beispielsweise Reaktionstemperaturen von etwa 160°C, und DMSO als Lösungsmittel eingesetzt wird. Dies läßt eine Umsetzung der Synthese auf kommerziell erhältliche Synthesemodule nur eingeschränkt zu. Des weiteren ist DMSO als hochsiedendes Lösungsmittel nur sehr schwer entfembar.

Die Verbindung 3-*N*-Boc-1-(3-*O*-nosyl-5-*O*-trityl-2-deoxy-β-D-lyxofuranosyl)thymin zur Herstellung von [¹⁸F]FLT ist beschrieben in Nuklearmedizin 2000; 39; 37-55, 8144; European Journal of Nuclear Medicine, 2000, Vol. 27, No. 8, S. 889-1276, BP27-BP30 und 47^{th} Annual Meeting of the Society of Nuclear Medicine, St. Louis, Missouri, 3. bis 7. Juni 2000, Abstract 1123, 255P. Allerdings ergibt diese Verbindung [¹⁸F]FLT nur in niedrigeren Ausbeuten.

Der vorliegenden Erfindung liegt also die Aufgabe zugrunde, ein Mittel und ein Verfahren zur Herstellung markierungsfähiger Verbindungen für die Synthese von [¹⁸F]FLT bereitzustellen, die nicht die aus dem Stand der Technik bekannten Nachteile aufweisen.

Erfindungsgemäß wird dies durch eine Verbindung erreicht, die sich dadurch auszeichnet, daß sie die Struktur der Formel (1) aufweist: 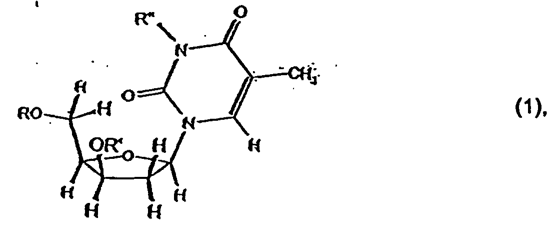 in der
R für am Phenyl-Rest substituiertes Triphenylmethyl oder am Phenyl-Rest substituiertes Triphenylsilyl;
R' für R¹-SO₂, wobei R¹ ein unsubstituiertes oder substituiertes C₁-C₅-Alkyl oder ein unsubstituiertes oder substituiertes Phenyl bedeutet, und
R" für ein C₂-C₁₀-Alkyloxycarbonyl stehen.

In der erfindungsgemäßen Verbindung ist im Triphenylmethyl- oder Triphenylsilyl-Rest der Phenyl-Rest substituiert. Der Triphenylmethyl- und Triphenylsilyl-Rest umfaßt jeweils drei Phenyl-Reste, so daß 1, 2 oder 3 der Phenyl-Reste substituiert sein können. Die Substituenten an den PhenylResten können dabei unabhängig voneinander gewählt sein, sie können aber auch gleich sein. An einem Phenyl-Rest kann 1 Substituent oder es können mehrere Substituenten vorliegen, die gleich oder verschieden voneinander sein können. Die Substituenten können sich in o-, m- und/oder p-Stellung zum Methylkohlenstoff- oder zum Si-Atom befinden. Als Substituenten werden solche Reste eingesetzt werden, die einen + M-Effekt bewirken. Sie sollten keine Reaktion mit funktionellen Gruppen der Edukte und Produkte eingehen, die bei der Herstellung und Weiterverarbeitung der erfindungsgemäßen Verbindung eingesetzt werden.

Bevorzugte Substituenten am Phenyl-Rest sind neben Methyl, Ethyl, Methoxy und Ethoxy, da letztere einen besonders günstigen + M-Effekt ausüben.

In einer bevorzugten Ausführungsform der erfindungsgemäßen Verbindung steht R für 4,4'-Dimethoxytriphenylmethyl, da es sich hierbei um eine sehr gute Abgangsgruppe handelt, die in besonders schonender und einfacher Weise, insbesondere bei milden Bedingungen (pH, niedrige Temperaturen), abgespalten werden kann, sowie ferner leicht herstellbar und in die erfindungsgemäße Verbindung leicht einfügbar ist. Die 4,4'-Dimethoxytriphenylmethyl-Gruppe kann in hoher Ausbeute bei milden Bedingungen, beispielsweise Raumtemperatur, an die OH-Gruppe in 5'-Position selektiv eingefügt werden. Des weiteren ergeben erfindungsgemäße Verbindungen mit Dimethoxytriphenylmethyl-Gruppen [¹⁸F]FLT in hohen Ausbeuten.

Gemäß der vorstehenden Formel (1) steht R' für R¹-SO₂, wobei R¹ ein unsubstituiertes oder substituiertes C₁-C₅-Alkyl sein kann. Beispiel eines unsubstituierten C₁-C₅-Alkyl-Restes ist Methyl, d. h. R¹-SO₂ steht für Methansulfonyl. Dieses ist bevorzugt, da die Mesylierung, d. h. die Reaktion, mit der es in die erfindungsgemäße Verbindung eingeführt wird, schnell abläuft und das Produkt in hohen Ausbeuten erhalten wird. Der Substituent am substituierten C₁-C₅-Alkyl ist vorzugsweise eine elektronenziehende Gruppe, damit der Rest R' eine gute Abgangsgruppe darstellt. Beispiele von elektronenziehenden Gruppen sind Halogene, wie F, Cl, Br und I, sowie NO₂, wobei wegen der günstigen elektronenziehenden Eigenschaft Fluor besonders geeignet ist. Beim substituierten C₁-C₅-Alkyl kann mindestens 1 bis alle der H-Atome des Alkyl-Restes durch einen elektronenziehenden Substituenten ersetzt sein. Ein besonders bevorzugter Vertreter des substituierten C₁-C₅-Alkyl-Rests ist CF₃, d. h. R¹-SO₂ steht für Trifluormethansulfonyl.

Wie bereits vorstehend ausgeführt wurde, kann der Rest R¹ ein unsubstiuiertes oder ein substituiertes Phenyl sein. Dabei kann 1 oder mehrere Substituent(en) vorliegen, die gleich oder voneinander verschieden sein können. Die Substituenten können sich in o-, m- und/oder p-Position zum SO₂-Rest befinden. Beispiele geeigneter Substituenten sind C₁-C₅-Alkyl-Reste, wie Methyl, oder elektronenziehende Substituenten, insbesondere Halogene, wie F, Cl, Br und I, sowie die NO₂-Gruppe.

Bevorzugte Reste R¹-SO₂ sind 4-Nitrophenylsulfonyl oder p-Toluensulfonyl.

Besonders günstige Vertreter für den in Formel (1) angegebenen C₂-C₁₀-Alkyloxycarbonyl-Rest ist *tert*-Butyloxycarbonyl und Neopentyloxycarbonyl. Diese sind besonders geeignet, da bei deren Abspaltung durch saure Hydrolyse CO₂ und ein Alkohol entstehen, die gut, nahezu vollständig und leicht vom Reaktionsgemisch abtrennbar sind.

Bevorzugte erfindungsgemäße Verbindungen sind 3-*N*-Boc-1-(5-*O*-(4,4'-dimethoxytrityl)-3-*O*-nosyl-2-deoxy-β-D-lyxofuranosyl)thymin, 3-*N*-Boc-1-(5-*O*-(4,4'-dimethoxytrityl)-3-*O*-tosyl-2-deoxy-β-D-lyxofuranosyl)thymin und 3-*N*-Boc-1-(5-*O*-(4,4'-dimethoxytrityl)-3-*O*-mesyl-2-deoxy-β-D-lyxofuranosyl)thymin.

Überraschenderweise wurde nun festgestellt, daß ausgehend von den erfindungsgemäßen Verbindungen in einfacher und schneller Weise die in der Positronen-Emissions-Tomographie verwendete Verbindung [¹⁸F]FLT hergestellt werden kann. Es war ferner vollkommen überraschend, daß die erfindungsgemäßen Verbindungen in einfacher und schneller Weise in guten Ausbeuten und hoher Reinheit unter milden Bedingungen hergestellt werden können.

Gegenstand der vorliegenden Erfindung ist ferner ein Verfahren zur Herstellung der erfindungsgemäßen Verbindung der Formel (1), wobei eine Verbindung der Formel (2) 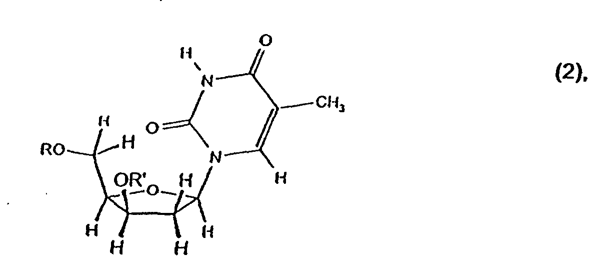 in der R und R' wie vorstehend definiert sind, mit Pyrokohlensäure-di-C₂-C₁₀-Alkylester umgesetzt wird.

Die Umsetzung eines solchen Esters, z. B. Pyrokohlensäure-di-*tert*-butylester (Boc₂O) mit einem N-Atom in einem Molekül ist dem Fachmann bekannt; er kennt hierzu notwendige Reaktionsbedingungen, Chemikalien und Geräte. Beispielsweise kann diese Umsetzung unter Inertgas und bei Raumtemperatur stattfinden. Als Lösungsmittel kann beispielsweise trockenes Pyridin eingesetzt werden. Die Isolierung und Reinigung des gewünschten Produktes, d. h. der erfindungsgemäßen Verbindung, kann in üblicher Weise erfolgen. Dazu kann beispielsweise das Lösungsmittel unter vermindertem Druck entfernt und das Rohprodukt chromatographisch, beispielsweise an Kieselgel, gereinigt werden.

Die Verbindung der Formel (2) kann vorzugsweise hergestellt werden durch Umsetzung der Verbindung der Formel (3) 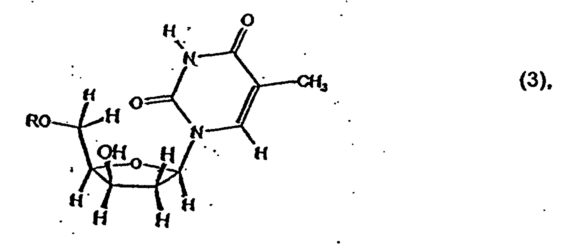 mit R¹-SO₂Hal, wobei R¹ wie vorstehend definiert ist und Hal für ein Halogen, insbesondere Chlor, steht, wodurch der Substituent R' eingeführt wird. Beispiele für R¹-SO₂Hal sind Methansulfonylchlorid, 4-Nitrophenylsulfonylchlorid, p-Toluensulfonylchlorid und Trifluormethansulfonsäurechlorid. Diese Umsetzung kann in günstiger Weise bei Raumtemperatur durchgeführt werden. Als Lösungsmittel kann trockenes Pyridin oder ein Gemisch von Triethylamin/Dichlormethan eingesetzt werden. Die Verbindung der Formel (3) kann in dem Lösungsmittel vorgelegt und die Verbindung der Formel R¹-SO₂Hal zugegeben werden. Die Isolierung und Reinigung des nach vorstehender Umsetzung erhaltenen Rohprodukts der Formel (2) kann beispielsweise durch Entfernen des Lösungsmittels unter vermindertem Druck und chromatographischer Reinigung, beispielsweise an Kieselgel, erfolgen.

Die Verbindung der Formel (3) kann - sofern sie nicht kommerziell erhältlich ist - ausgehend von Thymidin durch Einführung der Schutzgruppe R und Konfigurationsumkehr hergestellt werden. Diese Reaktionen sind dem Fachmann bekannt, d. h. er kennt hierzu notwendige Reaktionsbedingungen, Chemikalien und Geräte.

Zur Einführung der Schutzgruppe R in das Thymidin-Molekül kann dieses beispielsweise in Pyridin gelöst und unter Inertgas mit einer Verbindung vom Typ R-Hal umgesetzt werden, wobei R wie vorstehend definiert ist und Hal für ein Halogen, insbesondere Chlor, steht. Beispiele von R-Hal sind Triphenylmethylchlorid und 4,4'-Dimethoxytriphenylmethylchlorid.

Ein Syntheseweg für die erfindungsgemäße Verbindung, ausgehend von Thymidin, ist zusammenfassend nachfolgend dargestellt, wobei das erfindungsgemäße Verfahren den letzten Schritt darstellt.

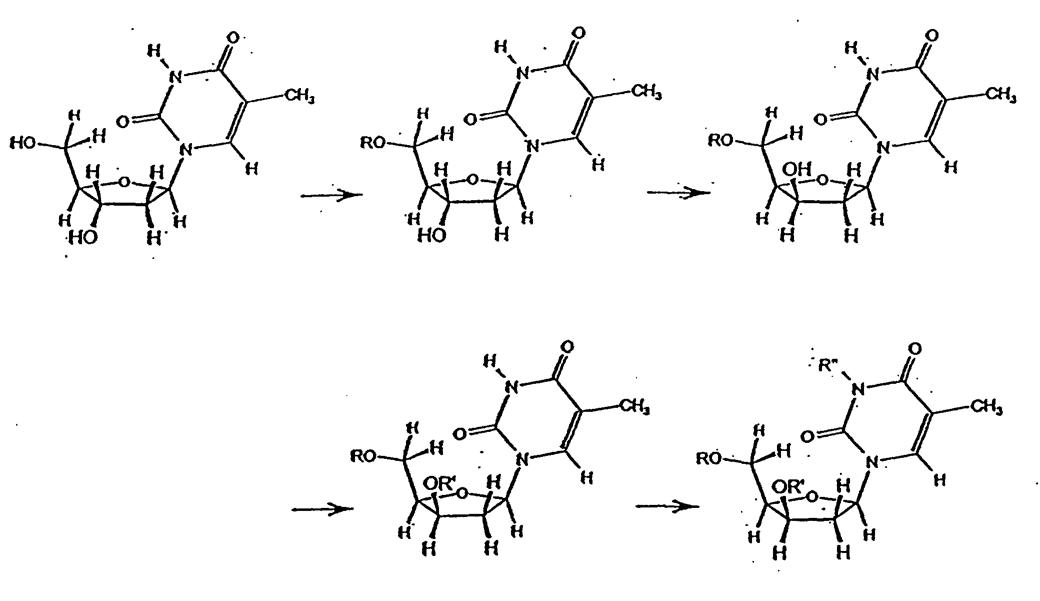

Dabei besitzen die Reste R, R' und R" die vorstehenden Bedeutungen.

Stehen kommerziell erhältliche Produkte zur Verfügung, die in vorstehendem Syntheseschema als Zwischenstufen aufgeführt sind, wie beispielsweise die Verbindung der Formel (3), in der R für Triphenylmethyl steht, kann die Synthese der erfindungsgemäßen Verbindung natürlich ausgehend von den kommerziellen Verbindungen beginnen.

Das erfindungsgemäße Verfahren weist eine Reihe von Vorteilen auf. So werden als Edukte bzw. Lösungsmittel handelsübliche Reagenzien verwendet, die schnell und preisgünstig zur Verfügung stehen. Des weiteren werden allgemein übliche Laborpraktiken eingesetzt, wie Umsetzung bei Raumtemperatur, Durchführung der Reaktionen mit üblichen Rührern, Verwendung von Inertgas und chromatographische Verfahren zur Isolierung und Reinigung von Produkten. Die Reaktionstemperatur beim erfindungsgemäßen Verfahren ist niedrig, so daß keine aufwendigen Vorrichtungen zum Erhitzen erforderlich sind. Die beim erfindungsgemäßen Verfahren eingesetzten Lösungsmittel sind im allgemeinen niedrig siedend und können somit leicht abgetrennt werden. Das erfindungsgemäße Verfahren ist automatisierbar, so daß es aus strahlenschutztechnischen Gründen günstig ist, da die Strahlenbelastung und die Kontamination von Menschen so gering wie möglich gehalten wird. Ausgehend von den mit dem erfindungsgemäßen Verfahren hergestellten erfindungsgemäßen Verbindungen kann in einfacher und schneller Weise das in der Positronen-Emissions-Tomographie verwendete [¹⁸F]FLT hergestellt werden, das in hoher Ausbeute und in hoher Reinheit erhältlich ist.

Gegenstand der vorliegenden Erfindung ist ferner die Verwendung der erfindungsgemäßen Verbindung zur Herstellung von [¹⁸F]FLT. Dabei wird vorzugsweise die OR'-Gruppe durch ¹⁸F substituiert. Die Schutzgruppen R" und R können dann abgespalten werden, vorzugsweise durch saure Hydrolyse.

Während der Markierungsreaktion kann die erfindungsgemäße Verbindung mit [¹⁸F]Fluorid in einem organischen Lösungsmittel, insbesondere Acetonitril, in Gegenwart einer Base, wie Kaliumcarbonat, und eines makrocyclischen Neutral-Liganden, wie Kryptofix® 222 der Firma Merck, zu dem R und R" geschützten [¹⁸F]FLT umgesetzt werden. Die Hydrolyse zur Zielverbindung [¹⁸F]FLT kann mit verdünnter Salzsäure durchgeführt werden. Da durch das Schutzgruppenmuster der erfindungsgemäßen Verbindung eine einfache saure Hydrolyse der [¹⁸F]-markierten Zwischenstufen in homogener Lösung mit verdünnter Salzsäure möglich ist, wird nach der Neutralisation lediglich untoxisches Natriumchlorid (Kochsalz) erhalten. Dieses kann mittels HPLC in einfacher Weise abgetrennt werden. Das Rohprodukt [¹⁸F]FLT kann chromatographisch gereinigt werden, z. B. mit einer Aluminiumoxid-Kartusche und/oder mittels HPLC (z. B. Laufmittel: H₂O:Ethanol = 92,5:7,5, isokratisch; Säule: Phenomenex LUNA 5 µ 250 x 4,6 mm).

Die Verwendung der Schutzgruppe Boc in der erfindungsgemäßen Verbindung, die zur Herstellung von [¹⁸F]FLT eingesetzt werden kann, weist den Vorteil auf, daß sie, im Gegensatz zu z. B. der Acylschutzgruppe, nicht oder, wenn überhaupt, nur unwesentlich zu einem Ausbeuteverlust während der [¹⁸F]FLT-Synthese führt.

Mit der erfindungsgemäßen Verwendung, d. h. die Verwendung der erfindungsgemäßen Verbindung zur Herstellung des Proliferationsmarkers [¹⁸F]FLT, ist es in überraschend einfacher und schneller Weise unter milden Bedingungen in guten Ausbeuten und in hoher Reinheit möglich, diesen Proliferationsmarker herzustellen. Dieses Herstellungsverfahren ist automatisierbar, d. h. es kann eine Vorrichtung konstruiert werden, mit der dieses Verfahren automatisch durchgeführt werden kann. Aus strahlenschutztechnischen Gründen ist dies bevorzugt, da die Strahlenbelastung und die Kontaminierung von Menschen bei einem vollautomatischen Verfahren niedrig ist. Da der Proliferationsmarker in hoher Reinheit erhältlich ist, wenn er ausgehend von der erfindungsgemäßen Verbindung synthetisiert wird, kann er dem Patienten entweder sofort oder ohne weitere aufwendige Reinigungsschritte verabreicht werden, ohne daß durch eventuelle Verunreinigungen unerwünschte Wirkungen beim Patienten auftreten. Aufgrund der hohen Ausbeute, in der der Proliferationsmarker erhältlich ist, können bei einer Radiosynthese ausreichende Menge an Marker hergestellt werden, um mehrere Patienten zu untersuchen.

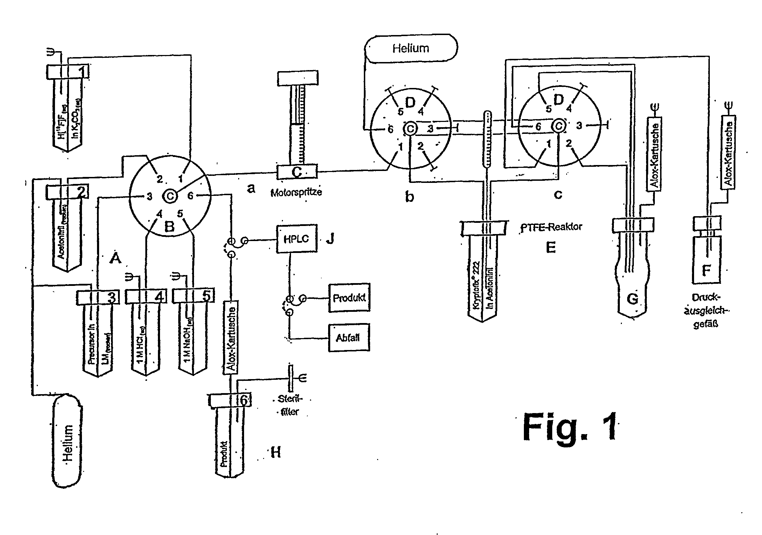

Die folgenden Beispiele erläutern die Erfindung näher, wobei
- Fig. 1: eine Vorrichtung zur Herstellung von [¹⁸F]FLT zeigt, wie sie in Beispiel 6 verwendet wird

### Beispiel 1: Synthese der erfindungsgemäßen Verbindung 3-N-Boc-1-(5-O-(4,4'-dimethoxytrityl)-3-O-mesyl-2-deoxy-β-D-lyxofuranosyl)thymin

### 1. Synthese von 5'-O-(4,4'-Dimethoxytrityl)thymidin

| | | | |
|---|---|---|---|
| **Ansatz** | 5,12 g | (21,14 mmol) | Thymidin |
| | 8,56 g | (25,26 mmol) | 4,4'-Dimethoxytriphenylmethylchlorid (Dimethoxytritylchlorid) |
| | 100 ml | | Pyridin (trocken) |

**Durchführung:** In einem 250 ml Dreihalskolben werden nacheinander Thymidin und Dimethoxytritylchlorid in trockenes Pyridin unter Inertgas eingetragen und 2 Stunden bei Raumtemperatur gerührt. Die DC-Reaktionskontrolle (*V*_{*MeOH:*}*V*_{*CH*_{*2*}*Cl*_{*2*}} = 1:19) zeigt als Hauptprodukt die 5'-*O*-geschützte Verbindung (*Rf* = 0,26) sowie je einen schwachen Fleck des Edukts (*Rf* = 0,00) und des zweifach geschützten Nebenprodukts 3',5'-Bis-*O*-(4,4-'-dimethoxytrityl)thymidin (*Rf* = 0,71). Danach wird die Reaktionslösung langsam in 1,5 I kräftig gerührtes Eiswasser gegossen. Nach 30 Minuten Rühren wird die gelbe Suspension abgesaugt und dem Festkörper die Restfeuchte am Rotationsverdampfer durch zweimalige azeotrope Destillation mit Ethanol entzogen. Das Rohprodukt wird alternativ aus einem Benzol-Aceton-Gemisch umkristallisiert oder mittels Säulenchomatographie (Kieselgel 60; Ø = 6 cm; *h* = 45 cm; *V*_{*MeOH:*}*V*_{*CH*_{*2*}*Cl*_{*2*}} = 1:19 + 0,1 % Triethylamin; auf ungefähr 20 g Kieselgel 60 aufgezogen) aufgereinigt und ergibt nach Trocknung im Hochvakuum das Produkt 5'-O-(4,4'-Dimethoxytrityl)thymidin.

| | | | |
|---|---|---|---|
| **Ausbeute** | 10,54 g | (19,35 mmol) | 91,5 % der Theorie |
| **Eigenschaften** | beigefarbener, amorpher Festkörper; Fp. = 114 - 116 °C (Sublimation) *Rf* = 0,23 (*V*_{*MeOH:*}*V*_{*CH*_{*2*}*Cl*_{*2*}}= 1:19) | | |

### 2. Synthese von 1-(5-O-(4,4'-Dimethoxytrityl)-2-deoxy-β-D-lyxofuranosyl)thymin

| | | | |
|---|---|---|---|
| **Ansatz** | 10,04 g | (18,44 mmol) | 5'-*O*-(4,4'-Dimethoxytrityl)thymidin |
| | 3,26 g | (28,46 mmol, 2,2 ml) | Methansufonylchlorid (Mesylchlorid) |
| | 4,82 g | (47,63 mmol, 6,6 ml) | Triethylamin |
| | 160 ml | | Tetrahydrofuran _{(getrocknet über Natrium; frisch destilliert)} |
| | 53 ml | | Ethanol |
| | 53 ml | | 1 M NaOH (aq) |
| | 37 ml | | 10 M NaOH (aq) |

**Durchführung:** In einem 500 ml Einhalskolben mit Gasansatz werden 5'-*O*-(4,4'-Dimethoxytrityl)-thymidin und Triethylamin unter Inertgas und Rühren in trockenem Tetrahydrofuran gelöst und auf eine Temperatur zwischen *t* ≈ -8 bis 0 °C (Eis/Ethanol) gekühlt. Zu der Reaktionslösung tropft man langsam Mesylchlorid und rührt 30 Minuten nach. Statt des Eduktflecks (*Rf* = 0,26) ist auf der DC-Platte (*V*_{*MeOH:*}*V*_{*CH*_{*2*}*Cl*_{*2*}} = 1:19) der Fleck der mesylierten Verbindung (*Rf* = 0,39) zu erkennen. Das Eisbad wird entfernt, der trüben, gelben Reaktionsmischung je 53 ml Wasser, Ethanol und 1 M Natronlauge zugefügt und 90 Minuten am Rückfluß gerührt. Auf dem DC (*V*_{*MeOH:*}*V*_{*CH*_{*2*}*Cl*_{*2*}} = 1:19) ist ausschließlich der Fleck der Anhydroverbindung (*Rf* = 0,20) sichtbar. Anschließend werden 37 ml 10 M Natronlauge zugegeben, wobei die intensiv-gelbe Reaktionslösung zwei Phasen ausbildet. Nach 45 Minuten Rühren bei einer Heizbadtemperatur von *t* ≈ 85 bis 90 °C läßt man die Reaktion bei Raumtemperatur während 16 Stunden vervollständigen. Die DC-Kontrolle (*V*_{*MeOH:*}*V*_{*CH*_{*2*}*Cl*_{*2*}} = 1:19) zeigt die Bildung des gewünschten Produkts (*Rf* = 0,31). Am Rotationsverdampfer wird das THF weitgehend aus dem Reaktionsgemisch entfernt, die zurückbleibende wäßrige Phase mit Wasser auf 400 ml verdünnt und dreimal mit je 400 ml Essigsäureethylester extrahiert. Die vereinigten organischen Phasen werden über Magnesiumsulfat getrocknet und das Solvens zunächst am Rotationsverdampfer, später im Hochvakuum entfernt, wobei das Produkt in ausreichender Reinheit erhalten wird. Die vollständige Abtrennung der Natronlauge kann durch Messung des pH-Werts einer Lösung des Produktes in Aceton/Wasser (*V*_{*Aceton*} : *V*_{*H*_{*2*}*O*} ≈ 1:1) bestätigt werden.

| | | | |
|---|---|---|---|
| **Ausbeute** | 9,32 g | (17,11 mmol) | 92,8 % der Theorie |
| **Eigenschaften** | farbloser, amorpher Festkörper; Rf= 0,31 (*V*_{*MeOH:*}*V*_{*CH*_{*2*}*Cl*_{*2*}} = 1:19) | | |

### 3. Synthese von 1-(5-O-(4,4'-Dimethoxytrityl)-3-O-mesyl-2-deoxy-β-D-lyxofuranosyl)thymin

| | | | |
|---|---|---|---|
| **Ansatz** | 1,97 g | (3,62 mmol) | 1-(5-*O*-(4,4'-Dimethoxytrityl)-2-deoxy-β-D- |
| | | | lyxofuranosyl)thymin |
| | 0,83 g | (7,25 mmol, 0,56 ml) | Methansulfonylchlorid (Mesylchlorid) |
| | 1,10 g | (10,82 mmol, 1,51 ml) | Triethylamin |
| | 25 ml | | Dichlormethan (trocken) |

**Durchführung:** In einem 50 ml Einhalskolben mit Gasansatz wird unter Inertgas und Rühren das 1-(5-*O*-(4,4'Dimethoxytrityl)-2-deoxy-β-D-lyxofuranosyl)thymin und das Triethylamin in trockenem Dichlormethan vorgelegt und bei *t* = 0 °C das Mesylchlorid langsam zugetropft. Nach 45 Minuten Rühren hat die gelblich-trübe Reaktionslösung Raumtemperatur erreicht. Bei der anschließenden DC-Kontrolle (*V*_{*MeOH:*}*V*_{*CH*_{*2*}*Cl*_{*2*}} = 1:19) ist kein Eduktfleck (*Rf* = 0,31) mehr sichtbar. Man fügt zirka 4 g Kieselgel 60 zu und entfernt das Lösungsmittel am Rotationsverdampfer. Das Produkt wird mittels Säulenchromatographie (Kieselgel 60; Ø = 4,5 cm, h = 25 cm; V_{MeOH:}V_{CH_{*2*}Cl_{*2*}} = 1:19 + 0,1 % Triethylamin) isoliert.

| | | | |
|---|---|---|---|
| **Ausbeute** | 1,83 g | (2,94 mmol) | 81,2 % der Theorie |
| **Eigenschaften** | farbloser, amorpher Festkörper; R*f* = 0,43 (V_{MeOH:}V_{CH₂Cl₂} = 1:19) | | |

### 4. Herstellung der erfindungsgemäßen Verbindung

| | | | |
|---|---|---|---|
| **Ansatz** | 1,65 g | (2,65 mmol) | 1-(5-*O*-(4,4'-Dimethoxytrityl)-3-*O*-mesyl-2-deoxy-β-D-lyxofuranosyl)thymin |
| | 3,52 g | (16,13 mmol, 3,7 ml) | Pyrokohlensäure-di-*tert*-butylester (Boc₂O) |
| | 30 ml | | Pyridin (trocken) |

**Durchführung:** In einem 100 ml Einhalskolben mit Gasansatz wird das 1-(5-*O*-(4,4'-Dimethoxytrityl)-3-*O*-mesyl-2-deoxy-β-D-lyxofuranosyl)thymin in trockenem Pyridin unter Inertgas vorgelegt, Boc₂O zugegeben und die Reaktionslösung bei Raumtemperatur gerührt. Nach mehrmaliger DC-Kontrolle (V_{Et₂O} = 100) innerhalb von 2 Tagen ist keine weitere Umsetzung mehr erkennbar. Neben dem Fleck des Edukts (R*f* = 0,07) wird ein Fleck (R*f* = 0,44) beobachtet, der eine Überlagerung eines nicht identifizierten Nebenprodukts (R*f* = 0,93, V_{MeOH:}V_{CH_{*2*}Cl_{*2*}} = 1:19) mit dem gewünschten Produkt (R*f* = 0,84; V_{MeOH:}V_{CH_{*2*}Cl_{*2*}} = 1:19, Merck® DC-Platten) darstellt. Der dunkelbraunen Reaktionslösung werden etwa 6 g Kieselgel 60 zugefügt und das Lösungsmittel zunächst am Rotationsverdampfer, danach 16 Stunden im Hochvakuum entfernt. Die Säulenchromatographie (Kieselgel 60; ∅ = 5 cm, h = 30 cm; V_{Et₂O} = 100 + 0,1 % Triethylamin) liefert das Titelprodukt als zweite Fraktion.

| | | | |
|---|---|---|---|
| **Ausbeute** | 0,32 g | (0,44 mmol) | 16,6% der Theorie |
| **Eigenschaften** | gelber, amorpher Festkörper; Fp. = 79,0 - 85,5 °C (Zersetzung); Rf = 0,44 (V_{Et2O} = 100) | | |

### Beispiel 2: Synthese der erfindungsgemäßen Verbindung 3-N-Boc-1-(5-O-(4,4'-dimethoxytrityl)-3-O-tosyl-2-deoxy-β-D-lyxofuranosyl)thymin

### 1. Synthese von 1-(5-O-(4,4'-Dimethoxytrityl)-3-O-tosyl-2-deoxy-β-D-lyxofuranosyl)thymin

| | | | |
|---|---|---|---|
| **Ansatz** | 2,00 g | (3,67 mmol) | 1-(5-*O*-(4,4'-Dimethoxytrityl)-2-deoxy-β-D-lyxofuranosyl)thymin, hergestellt nach Beispiel 3 |
| | 3,50 g | (18,36 mmol) | *p*-Toluensulfonylchlorid (Tosylchlorid) |
| | 30 ml | | Pyridin (trocken) |

**Durchführung**: In einem 100 ml Dreihalskolben mit Gasansatz werden in trockenem Pyridin bei Raumtemperatur unter Inertgas und Rühren nacheinander 1-(5-*O*-(4,4'-Dimethoxytrityl)-2-deoxy-β-D-lyxofuranosyl)thymin und Tosylchlorid gelöst und 7 Tage gerührt. In der DC-Reaktionskontrolle (V_{MeOH}:V_{CH₂Cl₂} = 1:19) läßt sich der Ablauf der Umsetzung nur bedingt beurteilen, da der Eduktfleck (R*f* = 0,31) von den Flecken des Tosylchlorids und des Pyridins überlagert wird. Über dem Produktfleck (R*f* = 0,50) sind mehrere Nebenproduktflecken zu erkennen. Zu der orangeroten Reaktionslösung gibt man zirka 5 g Kieselgel 60 und entfernt das Lösungsmittel zunächst am Rotationsverdampfer, später 16 Stunden im Hochvakuum. Die Titelverbindung wird säulenchromatographisch (Kieselgel 60; ∅ = 4,5 cm; *h* = 33 cm; V_{MeOH}:V_{CH₂Cl₂} = 1:19+ 0,1% Trietylamin) gereinigt.

| | | | |
|---|---|---|---|
| **Ausbeute** | 0,83 g | (1,19 mmol) | 32,4% der Theorie |
| **Eigenschaften** | beigefarbener, amorpher Festkörper; R*f* = 0,50 (V_{MeOH}:V_{CH₂Cl₂} = 1:19) | | |

### 2. Synthese der erfindungsgemäßen Verbindung

| | | | |
|---|---|---|---|
| **Ansatz** | 0,70 g | (1,00 mmol) | 1-(5-*O*-(4,4'-Dimethoxytrityl)-3-*O*-tosyl-2-deoxy-β-D-lyxofuranosyl)thymin |
| | 0,67 g | (3,07 mmol, 0,71 ml) | Pyrokohlensäure-di-*tert*-butylester (Boc₂O) |
| | 25 ml | | Pyridin (trocken) |

**Durchführung:** In einem 50 ml Einhalskolben mit Seitenhahn wird 1-(5-*O*-(4,4'-Dimethoxytrityl)-3-*O*-tosyl-2-deoxy-β-D-lyxofuranosyl)thymin bei Raumtemperatur unter Inertgas und Rühren in trockenem Pyridin gelöst, mit Boc₂O versetzt und 6 Tage gerührt. Auf dem DC (V_{Et₂O} = 100) sind sowohl der Edukt- (R*f* = 0,21) als auch der Produktfleck (R*f* = 0,67) sichtbar. Der braunen Reaktionslösung werden 5 g Kieselgel 60 zugegeben, das Pyridin zunächst am Rotationsdampfer und danach 16 Stunden im Hochvakuum entfernt. Die Säulenchromatographie (Kieselgel 60; ∅ = 4,0 cm; *h* = 30 cm; V_{Et₂O} = 100 + 0,1% Trietylamin) liefert das Produkt.

| | | | |
|---|---|---|---|
| **Ausbeute** | 0,50 g | (0,63 mmol) | 63,0% der Theorie |
| **Eigenschaften** | blaßgelber, amorpher Festkörper; Fp. = 87,0 - 93,0 °C R*f* = 0,67 ( V_{Et₂O} = 100) | | |

### Beispiel 3: Synthese der erfindungsgemäßen Verbindung 3-N-Boc-1-(5-O-(4,4'-dimethoxytrityl)-3-O-nosyl-2-deoxy-β-D-lyxofuranosyl)thymin

### 1. Synthese von 1-(3-O-Nosyl-5-O-(4,4'-dimethoxytrityl)-2-deoxy-β-D-lyxofuranosyl)thymin

| | | |
|---|---|---|
| **Ansatz:** | 0,58 g (1,07 mmol) | 1-(5-*O*-(4,4'-Dimethoxytrityl)-2-deoxy-β-D-lxyofuranosyl)thymin, hergestellt nach Beispiel 3 |
| | 0,72 g (3,25 mmol) | 4-Nitrophenylsulfonylchlorid (Nosylchlorid) |
| | 15 ml | Pyridin (trocken) |

**Durchführung:** In einem 25 ml Einhalskolben mit Gasansatz werden das 1-(5-*O*-(4,4'-Dimethoxytrityl)-2-deoxy-β-D-lxyofuranosyl)thymin und Nosylchlorid bei Raumtemperatur unter Inertgas und Rühren in trockenes Pyridin eingebracht und 6 Tage gerührt. Bei der anschließenden DC-Kontrolle (V_{MeOH}:V_{CH₂Cl₂} = 1:19) ist nur noch ein schwacher Eduktfleck (R_{f} = 0,31) sichtbar. Die braune Reaktionslösung wird mit zirka 3 g Kieselgel 60 versetzt, das Pyridin zunächst am Rotationsverdampfer, anschließend 16 Stunden im Hochvakuum entfernt. Man isoliert das Produkt mittels Säulenchromatographie (Kieselgel 60; ∅ = 2,9 cm; h = 36 cm; V_{MeOH}:V_{CH₂Cl₂} = 1:19 + 0,1 % Triethylamin).

| | | | |
|---|---|---|---|
| **Ausbeute** | 0,48 g | (0,66 mmol) | 61,7% der Theorie |
| **Eigenschaften:** | blaßgelber, amorpher Festkörper; Fp. = 85,9 - 93,4 °C; R_{f} = 0,53 (V_{MeOH}:V_{CH₂Cl₂} = 1:19) | | |

### 2. Herstellung der erfindungsgemäßen Verbindung

| | | | |
|---|---|---|---|
| **Ansatz** | 0,57 g | (0,78 mmol) | 1-(5-*O*-(4,4'-Dimethoxytrityl)-3-*O*-nosyl-2-deoxy-β-D-lxyofuranosyl)thymin |
| | 0,51 g | (2,34 mmol, 0,54 ml) | Pyrokohlensäure-di-*tert*-butylester (Boc₂O) |
| | 15 ml | | Pyridin (trocken) |

**Durchführung:** In einem 25 ml Einhalskolben mit Gasansatz legt man bei Raumtemperatur unter Inertgas und Rühren 1-(5-*O*-(4,4'-Dimethoxytrityl)-3-*O*-nosyl-2-deoxy-β-D-lxyofuranosyl)thymin in trockenem Pyridin vor und tropft Boc₂O zu. Nach 2½ Tagen ist in der DC-Kontrolle (V_{Et₂O} = 100) statt des Eduktflecks (R_{f} = 0,19) ein Produktfleck (R_{f} = 0,62) sichtbar. Zu der orangebraunen Reaktionslösung werden 3 g Kieselgel 60 gegeben und das Lösungsmittel zuerst am Rotationsverdampfer und danach 16 Stunden im Hochvakuum entfernt. Das Produkt wird säulenchromatographisch (Kieselgel 60; ∅ = 2,9 cm; h = 33 cm; V_{Et₂O} = 100 + 0,1 % Triethylamin) isoliert.

| | | | |
|---|---|---|---|
| **Ausbeute** | 0,38 g | (0,46 mmol) | 58,9 % der Theorie |
| **Eigenschaften** | blaßgelber, amorpher Festkörper; Fp. = 118,8 -120,6 °C; R_{f} = 0,62 ( V_{Et₂O} = 100) | | |

### Beispiel 4: Synthese von 3'-[¹⁸F]-Fluor-3'-deoxythymidin ([¹⁸F]FLT)

| | | | |
|---|---|---|---|
| **Ansatz** | | 0,013 mmol | Markierungsvorläufer gemäß Beispielen 1-5 |
| | 7,8 mg | (0,021 mmol) | Kryptofix®222 (Macrocyclischer Neutral-Ligand für die Synthese der Fa. Merck) |
| | 2,3 ml | | Acetonitril (trocken; für die DNA-Synthese) |
| | 0,15 ml | | 1 M Salzsäure |
| | 0,15 ml | | 1 M Natronlauge |
| | 0,2 ml | (0,020 mmol k^{⊕}) | 0,05 M Kaliumcarbonat (aq) |
| | 15392 MBq | (416mCi) | [¹⁸F]Fluorid zum Zeitpunkt *T*₀ |

**Durchführung:** Die Synthese erfolgt in nachfolgend beschriebener und in Fig. 1 gezeigter Vorrichtung, die sich aus Gründen des Strahlenschutzes in einer Bleiburg befindet. Alle erforderlichen Manipulationen und die HPLC können von außen gesteuert werden. Das Doppel-6-Wege-Motorventil D wird synchron in die entsprechenden Stellungen eingeschaltet. Somit sind die Transportwege für Lösungen und Gase vorgegeben.

Das jeweilige Reagens läßt sich aus einem der Vorlagegefäße **A** über das 6-Wege-Motorventil **B** durch die **Leitung a** in die programmierbare Motorspritze **C** überführen. Nach einem Zwischenschritt zum Druckausgleich innerhalb der Spritze wird das Reagens durch die **Leitung b** und das Doppel-6-Wege-Motorventil **D** (Stellung **1)** in den Reaktor E befördert, der sich in einem (nicht gezeigten) Heizbad befindet. Der Druckausgleich innerhalb des Reaktors erfolgt über die **Leitung c,** die über das Ventil **D** (Stellung **1)** in ein Druckausgleichgefäß F führt. Die Entlüftung von **F** findet über eine Alox-Kartusche statt, die eine Kontamination der Umgebung mit [18F]Fluorwasserstoff oder einem kontaminierten Aerosol verhindert. Jeder Transportschritt z. B. auf einer Vorlage in den Reaktor wird einmal wiederholt, um Lösungsreste aus den Kapillaren zu entfemen.
Die azeotrope Trocknung des Kryptofix®-Kalium[¹⁸F]fluorid-Komplexes erfolgt in der Stellung **6** des Ventils **D.** Der Inertgasstrom wird hierbei im Reaktor **E** durch die siedende Reaktionslösung geleitet und das Azeotrop über die **Leitung c** in die Kühlfalle **G** überführt. Die Kühlfalle wird ebenfalls über eine Alox-Kartusche entlüftet.

Zu Beginn der Synthese bzw. der Hydrolyse wird das Ventil **D** während der Aufheizphase der Reaktionslösung in Stellung **2** gebracht. Hierbei erfolgt der Druckausgleich des Reaktorgasraums **E** über die Kapillare c und die mit Wasser auf Raumtemperatur temperierte Kühlfalle **G.** Stellung **3** des Ventils **D** schließt den Reaktor gasdicht ab und ermöglicht Reaktionstemperaturen oberhalb des Siedepunkts des Solvens. Durch den Druckanstieg innerhalb des Reaktors **E** werden je nach Lösungsmittelmenge bis zu 30% des Reaktionsansatzes in die bis zum Reaktorboden reichende Kapillare **b** gedrückt. Durch eine kurze Absenkung der Reaktionstemperatur unter den Siedepunkt des Solvens wird die Reaktionsmischung aus der Kapillare wieder in den Reaktor überführt. Nach der Abkühlung des Reaktionsgefäßes am Ende der Synthese bzw. der Hydrolyse erfolgt ein Druckausgleich in Stellung **5** des Ventils **D.**
Zum Abschluß der Synthese wird die Reaktionslösung aus dem Reaktor **E** über das Ventil **D** (Stellung **1)** in die Motorspritze **C** gezogen und von dort Ober das Ventil **B** (Stellung **6)** wahlweise in ein Produktgläschen **H** gedrückt, das mit einer Bleiabschirmung versehen ist, oder auf die Schleife der HPLC **J** aufgegeben. Nach der präparativen Trennung wird das Reinprodukt in einer Sammelstation in Produktgläschen fraktioniert, wobei die übrigen Fraktionen in ein Abfallgefäß abgetrennt werden.

Zur Herstellung von [¹⁸F]FLT befüllt man den Reaktor mit einer Lösung aus Kryptofix® in 1 ml trockenem Acetonitril, das Vorlagegefäß **2** mit **1** ml Acetonitril zur azeotropen Trocknung, das Vorlagegefäß **3** mit einer Lösung des Markierungsvorläufers (Precursors) in 300 µl trockenem Acetonitril und die Vorlagegefäße **4** und **5** mit je 150 µl 1 M Salzsäure zur Hydrolyse bzw. 1 M Natronlauge zur Neutralisation der Reaktionslösung. Ein Spitzbodenglas mit Septum und Belüftungskanüle, das sich in einer Bleiabschirmung befindet, wird mit der [¹⁸F]Fluorid-haltigen, wäßrigen Kaliumcarbonatiösung befüllt und an der Apparatur als Vorlagegefäß **1** angeschlossen, das mit einer Bleiabschirmung versehen ist, nachdem man die Aktivität *A*_{0A} zur Zeit T₀ bestimmt hat. Im ersten Schritt wird die basische [¹⁸F]Fluoridlösung in den Reaktor überführt, anschließend die Restaktivität im Spitzbodenglas bestimmt und das sich im Reaktor befindende Azeotrop im Helimstrom bei einer Heizbadtemperatur von *t* ≈ 115°C abdestilliert. Etwa 14 Minuten nach *T*₀ erreicht die Innentemperatur des Reaktors *t* ≥ 110°C. Nach 2 Minuten läßt man den Reaktor auf *t* ≤ 90°C abkühlen, befüllt diesen mit **1** ml Acetonitril und wiederholt den azeotropen Trocknungsschritt. Zirka **1** Minute nachdem sich die Reaktorinnentemperatur erneut auf *t* ≥ 110°C eingestellt hat, läßt man auf t ≤ 75°C abkühlen und überführt die Lösung des Markierungsvorläufers in Acetonitril in den Reaktor. Etwa 28 Minuten nach *T*₀ beginnt die Synthese der [¹⁸F]Fluor-markierten Zwischenstufe. Die Synthesezeit beträgt 10 Minuten bei einer Heizbadtemperatur von *t* ≈ 115°C unter Reaktorabschluß. Nachdem die Temperatur der Reaktionslösung *t* = 105°C überschritten hat - dies wird etwa 3 Minuten nach Synthesebeginn erreicht - läßt man die Reaktorinnentemperatur innerhalb 3 Minuten auf *t* ≤ 80°C absinken und erhitzt anschließend bis zum Ende der Synthesezeit auf *t* ≈ 110°C. Nach Abkühlung auf *t* ≤ 75°C wird der Reaktionslösung Salzsäure zugesetzt. Ungefähr 42 Minuten nach *T*₀ beginnt die Hydrolyse der markierten Zwischenstufe zu [¹⁸F]FLT, wobei hierfür die Arbeitsschritte der Synthese der Zwischenstufe wiederholt werden. Die auf *t* ≤ 75°C abgekühlte, orangebraune, schwach trübe Reaktionslösung wird mit Natronlauge neutralisiert, etwa 20 Sekunden gerührt und in die Aufgabenschleife der HPLC überführt. Der Start des HPLC-Laufs erfolgt etwa 58 Minuten nach *T*_{*0*}o. Das [¹⁸F]FLT wird bei einer Retentionszeit von etwa *T*_{R} ≈ 34 bis 41 min in einer wäßrig-ethanolischen Lösung (*V*_{EtOH}:*V*_{H2O}=7,5:92,5) in hoher Reinheit eluiert (HPLC-Säule Phenomenex; LUNA: 5µm; 21 x 250 mm; isokratisch; 10 ^{ml}/ₘᵢₙ).

| | | | | |
|---|---|---|---|---|
| **Ausbeute** | 1106 | MBq | (29.9 mCi) | 9,3% der Theorie; unkorrigiert; gemessen zum Zeitpunkt *T* = 97:20 min nach *T*₀ |
| | 2042 | MBq | (55,2 mCi) | 17,2% der Theorie; korrigiert auf Zeitpunkt *T*₀; |

Die prozentuale Ausbeute bezieht sich auf eine Anfangsaktivität von A₀ = 11892 MBq, die man erhält, wenn von der im Ansatz beschriebenen Anfangsaktivität A_{0A} = 15392 MBq die im Spitzglas verbliebene Restaktivität A_{0R} = 1228 MBq und der auf T₀ korrigierte Aktivitätsverlust in die Kühlfalle A_{0K} = 2272 MBq abgezogen wird. Die Berücksichtigung von A_{0K} ist nur dann notwendig, wenn sich eine ungewöhnlich hohe Aktivität am Ende der Synthese in der Kühlfalle befindet. Ursache hierfür kann z. B. eine Verstopfung im Abluftsystem der Apparatur und die daraus resultierenden Siedeverzüge während der azeotropen Trocknung sein.

**Eigenschaften:** farblose, klare, ethanolisch-wäßrige Lösung; pH 5,5; [¹⁸F]Fluorid-frei;
spezifische Aktivität: 33 ^{MBq}/ₘₗ(0,88^{mCi}/ₘₗ)

## Patentansprüche

1. Verbindung der Formel (1) 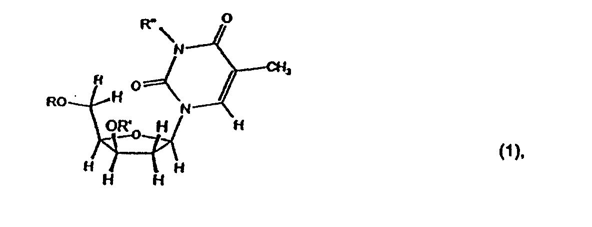 in der
R für am Phenyl-Rest substituiertes Triphenylmethyl oder am Phenyl-Rest substituiertes Triphenylsilyl, wobei als Substituenten solche Reste eingesetzt werden, die einen +M-Effekt bewirken;
R' für R¹-SO₂, wobei R¹ ein unsubstituiertes oder substituiertes C₁-C₅-Alkyl oder ein unsubstituiertes oder substituiertes Phenyl bedeutet, und
R" für ein C₂-C₁₀-Alkyloxycarbonyl stehen.

2. Verbindung nach Anspruch 1, wobei der Substituent am Phenyl-Rest unter Methyl, Ethyl, Methoxy und Ethoxy ausgewählt ist.

3. Verbindung nach Anspruch 1 oder 2, wobei R für 4,4'-Dimethoxytriphenylmethyl steht.

4. Verbindung nach einem der vorhergehenden Ansprüche, wobei R¹-SO₂ für Methansulfonyl, 4-Nitrophenylsulfonyl, p-Toluensulfonyl oder Trifluormethansulfonyl steht.

5. Verbindung nach einem der vorhergehenden Ansprüche, wobei das C₂-C₁₀-Alkyloxycarbonyl tert-Butyloxycarbonyl oder Neopentyloxycarbonyl ist.

6. Verbindung nach einem der vorhergehenden Ansprüche, die ausgewählt ist unter 3-N-Boc-1-(5-O-(4,4'-dimethoxytrityl)-3-O-nosyl-2-deoxy-β-D-lyxofuranosyl)thymin, 3-N-Boc-1-(5-O-(4,4'-dimethoxytrityl)-3-O-tosyl-2-deoxy-β-D-lyxofuranosyl)thymin und 3-N-Boc-1-(5-O-(4,4'-dimethoxytrityl)-3-O-mesyl-2-deoxy-β-D-lyxofuranosyl)thymin.

7. Verfahren zur Herstellung der Verbindung nach einem der Ansprüche 1 bis 6, wobei eine Verbindung der Formel (2) 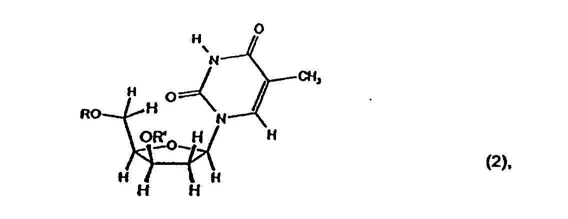 in der R und R' wie vorstehend definiert sind, mit Pyrokohlensäure-di-C₂-C₁₀-Alkylester umgesetzt wird.

8. Verfahren nach Anspruch 7, wobei die Verbindung der Formel (2) erhalten wird durch Umsetzen der Verbindung der Formel (3) 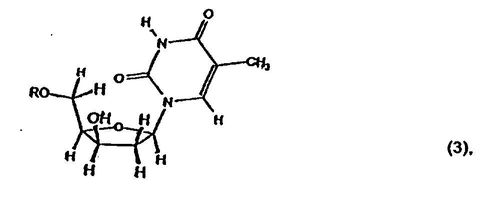 mit R¹-SO₂Hal, wobei R¹ wie vorstehend definiert ist und Hal für ein Halogen steht, wodurch der Substituent R' eingeführt wird.

9. Verfahren nach einem der Ansprüche 7 oder 8, wobei die Verbindung der Formel (3) aus Thymidin durch Einführen des Substituenten R und Konfigurationsumkehr hergestellt wird.

10. Verwendung der Verbindung nach einem der Ansprüche 1 bis 6 zur Herstellung von 3'-[¹⁸F]Fluor-3'-deoxythymidin.

11. Verwendung nach Anspruch 10, wobei die OR'-Gruppe durch ¹⁸F substituiert und die Schutzgruppen R" und R abgespalten werden.

## Claims

1. Compound of formula (1): 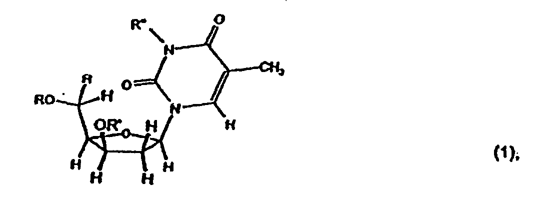 in which
R is triphenylmethyl substituted on the phenyl radical or triphenylsilyl substituted on the phenyl radical, the substituents used being radicals with a +M effect;
R' is R¹-SO₂, R¹ being an unsubstituted or substituted C₁-C₅-alkyl or an unsubstituted or substituted phenyl; and
R" is a C₂-C₁₀-alkoxycarbonyl.

2. Compound according to Claim 1 wherein the substituent on the phenyl radical is selected from methyl, ethyl, methoxy and ethoxy.

3. Compound according to Claim 1 or 2 wherein R is 4.4'-dimethoxytriphenylmethyl.

4. Compound according to one of the preceding claims wherein R¹-SO₂ is methanesulfonyl, 4-nitrophenylsulfonyl, p-toluenesulfonyl or trifluoromethanesulfonyl.

5. Compound according to one of the preceding claims wherein the C₂-C₁₀-alkoxycarbonyl is tert-butoxycarbonyl or neopentoxycarbonyl.

6. Compound according to one of the preceding claims which is selected from 3-N-Boc-1-(5-O-(4,4'-dimethoxytrityl)-3-O-nosyl-2-deoxy-β-D-lyxofuranosyl)-thymine, 3-N-Boc-1-(5-O-(4,4'-dimethoxytrityl)-3-O-tosyl-2-deoxy-β-D-lyxofuranosyl)thymine and 3-N-Boc-1-(5-O-(4,4'-dimethoxytrityl)-3-O-mesyl-2-deoxy-β-D-lyxofuranosyl)thymine.

7. Process for the preparation of the compound according to one of Claims 1 to 6, wherein a compound of formula (2): 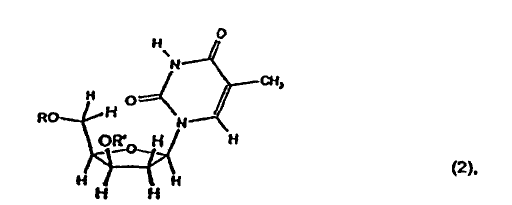 in which R and R' are as defined above, is reacted with di-C₂-C₁₀-alkyl pyrocarbonate.

8. Process according to Claim 7 wherein the compound of formula (2) is obtained by reacting the compound of formula (3): 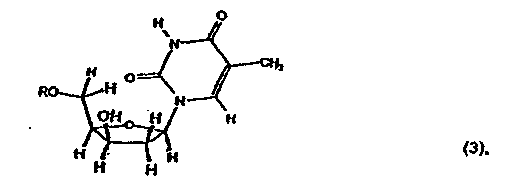 with R¹-SO₂Hal, in which R¹ is as defined above and Hal is a halogen, whereby the substituent R' is introduced.

9. Process according to Claim 7 or 8 wherein the compound of formula (3) is prepared from thymidine by introduction of the substituent R and inversion of configuration.

10. Use of the compound according to one of Claims 1 to 6 for the preparation of 3'-[¹⁸F]fluoro-3'-deoxythymidine.

11. Use according to Claim 10 wherein the OR' group is substituted by ¹⁸F and the protecting groups R" and R are cleaved.

## Revendications

1. Composé de formule (1) : 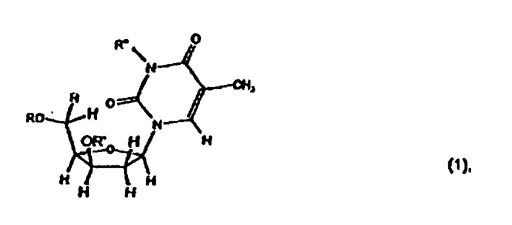 dans laquelle:
R représente un radical triphénylméthyle substitué sur les restes phényle ou un radical triphénylsilyle substitué sur les restes phényle, où comme substituant, on met en oeuvre des restes qui effectuent un effet +M ;
R' représente R¹-SO₂ où R¹ signifie un radical alkyle en C₁-C₅ non substitué ou substitué ou un radical phényle non substitué ou substitué, et
R" représente un radical alkyloxycarbonyle en C₂-C₁₀.

2. Composé selon la revendication 1, où le substituant sur les restes phényle est choisi parmi les groupes méthyle, éthyle, méthoxy et éthoxy.

3. Composé selon la revendication 1 ou 2, où R représente un groupe 4,4'-diméthoxytriphénylméthyle.

4. Composé selon l'une quelconque des revendications précédentes, où R¹-SO₂ représente un groupe méthanesulfonyle, 4-nitrophénylsulfonyle, p-toluènesulfonyle ou trifluorométhanesulfonyle.

5. Composé selon l'une quelconque des revendications précédentes, où le radical alkyloxycarbonyle en C₂-C₁₀ est un groupe t-butyloxycarbonyle ou néopentyloxycarbonyle.

6. Composé selon l'une quelconque des revendications précédentes, qui est choisi parmi la 3-N-Boc-1-(5-O-(4,4'-diméthoxytrityl)-3-O-nosyl-2-désoxy-β-D-lyxofurannosyl)thymine, la 3-N-Boc-1-(5-O-(4,4'-diméthoxytrityl)-3-O-tosyl-2-désoxy-β-D-lyxofurannosyl)thymine et la 3-N-Boc-1-(5-O-(4,4'-diméthoxytrityl)-3-O-mésyl-2-désoxy-β-D-lyxofurannosyl) thymine.

7. Procédé de préparation du composé selon l'une quelconque des revendications 1 à 6, où un composé de formule (2) : 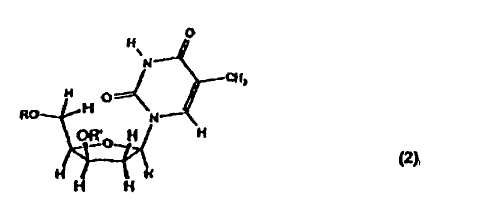 dans laquelle R et R' sont tels que définis précédemment, est mis à réagir avec un ester di(alkylique en C₂-C₁₀) de l'acide pyrocarbonique.

8. Procédé selon la revendication 7, où le composé de formule (2) est obtenu par réaction du composé de formule (3) : 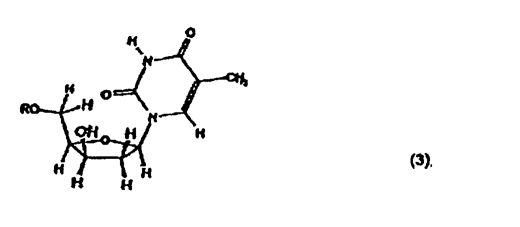 avec R¹-SO₂Hal, où R¹ est tel que défini précédemment et Hal représente un halogène, ce par quoi on introduit le substituant R'.

9. Procédé selon l'une quelconque des revendications 7 et 8, où le composé de formule (3) est préparé à partir de la thymine, par introduction du substituant R et inversion de la configuration.

10. Utilisation du composé selon l'une quelconque des revendications 1 à 6, pour la préparation de la 3'-[¹⁸F]-fluoro-3'-désoxythymidine.

11. Utilisation selon la revendication 10, où le groupe OR' est substitué par ¹⁸F et les groupes protecteurs R" et R sont clivés.
